# EUROPÄISCHE PATENTSCHRIFT

(11) **EP 0 596 957 B1**
(45) Veröffentlichungstag und Bekanntmachung des Hinweises auf die Patenterteilung: **16.10.1996**
(21) Anmeldenummer: 92916262.6
(22) Anmeldetag: 27.07.1992
(51) Int. Cl.: C11D 1/37, C11D 1/14, C11D 17/00

(54) **FLIESSFÄHIGE WÄSSRIGE ALKYLSULFATPASTEN**
FREE-FLOWING AQUEOUS ALKYL SULPHATE SLURRIES
PATES AQUEUSES FLUIDES DE SULFATE D'ALKYLE

(30) Priorität: 03.08.1991 DE 4125792
(43) Veröffentlichungstag der Anmeldung: 18.05.1994
(73) Patentinhaber: Henkel Kommanditgesellschaft auf Aktien, 40191 Düsseldorf (DE)
(72) Erfinder: HOFMANN, Rainer, D-4000 Düsseldorf 13 (DE); GRUBER, Bert, D-5012 Bedburg (DE); SYLDATH, Andreas, D-4000 Düsseldorf (DE); KISCHKEL, Ditmar, D-4019 Monheim 2 (DE)
(86) Internationale Anmeldenummer: EP9201704
(87) Internationale Veröffentlichungsnummer: WO9303128

(56) Entgegenhaltungen:
- WO-A-91/02045
- WO-A-92/07054
- WO-A-92/16606
- FR-A- 2 371 509
- GB-A- 406 565

## Beschreibung

Die Erfindung betrifft ein Verfahren zur Herstellung von Alkylsulfatpasten mit verbesserten Fließeigenschaften durch Zusatz von Alkenylsulfaten.

Anionische Tenside vom Typ der Alkylsulfate, insbesondere solche, die Alkylreste mit 16 bis 18 Kohlenstoffatomen enthalten, zeigen ausgezeichnete Detergenseigenschaften und finden sowohl in flüssigen als auch in pulverförmigen Wasch- und Reinigungsmitteln Verwendung.

Zur Herstellung derartiger Mittel geht man in der Regel von wäßrigen Alkylsulfatpasten aus. Um einen Überflüssigen Massentransport zu vermeiden, ist es vorteilhaft, wenn wäßrige Tensidpasten einen möglichst hohen Feststoffgehalt aufweisen. Alkylsulfatpasten können jedoch nur bis zu einem bestimmten Feststoffgehalt aufkonzentriert werden. Darüber hinaus weisen derartige Pasten ein nicht-Newton'sches Fließverhalten auf. Oberhalb einer Grenze von etwa 55 Gew.-% Feststoffgehalt erreicht die Viskosität in der Regel so hohe Werte, daß die Pumpbarkeit der Tensidlösungen selbst bei erhöhten Temperaturen nicht mehr gewährleistet ist. So weisen wäßrige Fettalkoholsulfat-Pasten mit Feststoffgehalten von 30 bis 70 Gew.-% schon bei Umgebungstemperatur so hohe Viskositäten und Fließgrenzen auf, daß ein Umfüllen oder Umpumpen oft unmöglich ist.

In der europäischen Patentschrift EP 024 711 wird vorgeschlagen, Polyalkyletherglykolsulfate als viskositätsmindernde Zusätze zu schwer beweglichen Tensidkonzentraten zu geben. Während derartige Verbindungen gut geeignet sind, die Viskosität von Alkylethersulfat-, Alkylarylethersulfat-, Alkylbenzolsulfonat- und Alkylarylsulfosuccinat-Lösungen zu senken, ist ihre Leistung in Gegenwart von Alkylsulfaten, insbesondere von linearen, primären Alkylsulfaten, oft nicht befriedigend.

Die nicht vorveröffentlichte Patentan meldung WO-A-92 166 06 offenbart hochkonzentrierte Fettalkoholpasten, die durch die Umsetzung von SO₃ mit ungesättigten und gesättigten Fettalkoholen erhalten werden.

Aus der deutschen Offenlegungsschrift DE 34 47 859 ist die Verwendung von Alkansulfonaten als Viskositätsregler für hochviskose Aniontensid-Konzentrate, insbesondere für Salze von α-Sulfofettsäureestern, bekannt.

Die deutsche Offenlegungsschrift DE 37 18 896 beschreibt die Verwendung alkoxylierter Alkohole als Viskositätsregler für hochviskose Alkylbenzolsulfonat-Konzentrate.

Überraschenderweise wurde nun gefunden, daß wäßrige Mischungen von ungesättigten und gesättigten Alkylsulfaten eine geringe Viskosität und eine niedrige Fließgrenze aufweisen, so daß das für Alkylsulfate auf Basis ausschließlich gesättigter Alkohole typische ungünstige Fließverhalten überwunden wird. Hierdurch ist es möglich, Alkylsulfat-Pasten mit hohem Feststoffgehalt herzustellen, die auch bei niedrigen Temperaturen und geringem Schergefälle fließ- und pumpfähig sind.

Gegenstand der Erfindung ist ein Verfahren zur Herstellung einer fließfähigen und pumpbaren wäßrigen Alkylsulfatpaste mit einem Feststoffgehalt von 50 Gew.-% bis 80 Gew.-%, insbesondere 60 Gew.-% bis 75 Gew.-%, welches dadurch gekennzeichnet ist, daß man eine wäßrige Paste, die 50 Gew.-% bis 80 Gew.-% eines Alkylsulfats der Formel I,

R¹-O-SO₃M¹ (I)

in der R¹ einen linearen oder verzweigtkettigen Alkylrest mit 12 bis 22 C-Atomen und M¹ ein Alkalimetall-, ammonium-, Alkylammonium- oder Hydroxyalkylammonium-Ion bedeuten, mit einem Alkenylsulfat der Formel II,

R²-O-SO₃M² (II)

in der R² einen Alkenylrest mit 16 bis 22 C-Atomen und 1, 2 oder 3 Doppel bindungen und M² ein Alkalimetall-, Ammnium-, Alkylammonium- oder Hydroxyalkylammonium-Ion bedeuten, im Gewichtsverhältnis Alkylsulfat gemäß Formel I zu Alkenylsulfat gemäß Formel II von 10:1 bis 1:3 mischt.

Vorzugsweise werden Alkylsulfat gemäß Formel I und Alkenylsulfat gemäß Formel II im Gewichtsverhältnis von Alkylsulfat zu Alkenylsulfat von 7:1 bis 1:2 miteinander vermischt.

Dabei geht man vorzugsweise derart vor, daß man eine wäßrige Paste, welche Alkenylsulfat gemäß Formel II enthält, in einer solchen Menge mit einer wäßrigen Paste, die Alkylsulfat gemäß Formel I enthält, mischt, daß die Summe von Alkylsulfat und Alkenylsulfat in der resultierenden wäßrigen Paste 45 Gew.-% bis 80 Gew.-%, insbesondere 50 Gew.-% bis 75 Gew.-% beträgt und die resultierende Paste 20 Gew.-% bis 55 Gew.-% insbesondere 25 Gew.-% bis 50 Gew.-% Wasser enthält.

Die Alkylsulfate gemäß Formel I sind bekannte anionische Tenside, die in der Regel durch Umsetzung von aliphatischen primären Alkoholen mit einem Sulfatierungsreagenz, beispielsweise Schwefeltrioxid oder Chlorsulfonsäure, hergestellt werden. Alkylsulfate, auf die sich das erfindungsgemäße Verfahren erstreckt, leiten sich von Fettalkoholen mit 12 bis 22 Kohlenstoffatomen ab. Typische Beispiele hierfür sind Laurylalkohol, Myristylalkohol, Cetylalkohol, Stearylalkohol oder Behenylalkohol. Eine besonders drastische Erniedrigung der Pastenviskosität wird bei Alkylsulfaten beobachtet, die sich von Alkoholen mit 12 bis 18, insbesondere 16 bis 18 Kohlenstoffatomen ableiten.

Die Alkylsulfate können sich auch von technischen Alkoholgemischen ableiten, wie sie z.B. bei der Hydrierung von technischen Fettsäureestergemischen natürlicher Herkunft oder von Aldehyden aus der Roelen'schen Oxosynthese anfallen. Bevorzugt sind hierbei Alkylsulfate auf der Basis von technischen Kokos- oder hydrierten Palmalkoholschnitten. Hierunter sind solche primären Fettalkohole zu verstehen, die im Durchschnitt die folgende C-Kettenverteilung aufweisen:

| Kokosalkohol | | hydrierter Palmalkohol |
|---|---|---|
| C₁₀: | 0 - 3 Gew.-% | |
| C₁₂: | 48 - 58 Gew.-% | |
| C₁₄: | 19 - 24 Gew.-% | 0 - 3 Gew.-% |
| C₁₆: | 9 - 12 Gew.-% | 45 - 55 Gew.-% |
| C₁₈: | 11 - 14 Gew.-% | 45 - 55 Gew.-% |
| C₂₀: | | 0 - 3 Gew.-% |

Die im Sinne der Erfindung den Alkylsulfat-Pasten zuzusetzenden Sulfatierungsprodukte von ungesättigten Alkoholen stellen Verbindungen dar, die man durch Umsetzung von ein-, zwei- oder dreifach ungesättigten Alkoholen mit Sulfatiermitteln, insbesondere gasförmigem Schwefeltrioxid sowie nachfolgender Neutralisation und Hydrolyse der entstandenen Reaktionsprodukte erhält. Ein Verfahren zur Herstellung derartiger Verbindungen ist beispielsweise von M. Morak und K. Audiova in Tenside Detergents 15 (1978), 299 beschrieben.

Da bei Einsatz ungesättigter Alkohole bei der Sulfatierung auch eine Anlagerung des Schwefeltrioxids an die Doppelbindung stattfinden kann, können in der Alkenylsulfat-Paste auch Stoffe enthalten sein, die eine innenständige Sulfonatgruppe beziehungsweise eine Sulfonat- und eine Sulfatgruppe enthalten. Der Anteil dieser innenständigen Sulfierprodukte kann üblicherweise 3 bis 20 Gew.-%, bezogen auf die Menge an Alkenylsulfat gemäß Formel II, betragen. Durch entsprechende Reinigungsoperationen kann diese Menge gewünschtenfalls auf 0 gesenkt werden.

Als typisches Beispiel für ungesättigte Alkohole, deren Sulfate der Formel II im Sinne der Erfindung als viskositätsniedrigende Komponente dienen können, kommen insbesondere Palmitoleylalkohol, Oleylalkohol, Elaidylalkohol, Petroselinylalkohol, Linoleylalkohol, Linolenylakohol, Gadoleylalkohol oder Erucylalkohol in Betracht. Bevorzugt ist der Einsatz von Oleylalkohol. Es können auch Sulfatierungsprodukte von technischen Gemischen der genannten ungesättigten Alkohole untereinander oder mit Anteilen von gesättigten Alkoholen entsprechender Kettenlänge in dem erfindungsgemäßen Verfahren eingesetzt werden. Derartige Pasten enthalten vorzugsweise nicht mehr als 70 Gew.-%, insbesondere 2 Gew.-% bis 60 Gew.-% gesättigtes Alkylsulfat.

Als weitere Bestandteile können sowohl die beiden Ausgangsstoff-Pasten als auch die erfindungsgemäß hergestellte Alkylsulfat-Paste geringe Mengen, in der Regel nicht über 10 Gew.-%, insbesondere 2 Gew.-% bis 5 Gew.-%, bezogen auf die jeweilige Paste, aus der Herstellung resultierende anorganische Salze, insbesondere Sulfate, enthalten, wie sie aus der Reaktion eventuell überschüssigen Sulfatierungsmittels mit der Neutralisationsbase entstehen.

Die erfindungsgemäß hergestellten hochkonzentrierten Alkylsulfat-Pasten weisen ausgezeichnete Detergenseigenschaften und eine hohe Kaltwasserlöslichkeit auf. Sie werden daher vorzugsweise zur Herstellung von pulverförmigen oder flüssigen Wasch,- Spül- und Reinigungsmitteln sowie kosmetischen Produkten, insbesondere Mitteln zur Haar- und Körperpflege verwendet.

Derartige Mittel können in einfacher Weise durch Verdünnen der nach dem erfindungsgemäßen Verfahren hergestellten Pasten mit Wasser auf die gewünschte Aktivsubstanzkonzentration hergestellt werden. Die Zugabe anderer in derartigen Mitteln üblicher Bestandteile, zu denen insbesondere Buildersubstanzen, wie Zeolithe und Schichtsilikate, Korrosionsinhibitoren, Bleichmittel, Bleichaktivatoren, optische Aufheller, Enzyme, Vergrauungsinhibitoren, antimikrobielle Wirkstoffe, wassermischbare Lösungsmittel, Abrasivmittel, Schaumstabilisatoren, Konservierungsmittel, pH-Regulatoren, Farb- und Duftstoffe sowie zusätzliche Tenside gehören, ist möglich.

### Beispiele

### Beispiel 1

In einem 1-l-Sulfierreaktor mit Mantelkühlung und Gaseinleitungsrohr wurden 260 g (1 mol) eines technischen Oleyl-Cetylalkohols (HD-Ocenol^{(R)} 50-55, Hersteller Henkel) der folgenden Zusammensetzung
- Myristylalkohol :: 5 Gew.-%
- Cetylalkohol :: 30 Gew.-%
- Oleylalkohol :: 65 Gew.-%
- Iodzahl :: 53
- Hydroxylzahl :: 215

vorgelegt und bei 45 °C mit 84 g (1,05 mol) Schwefeltrioxid umgesetzt. Das Schwefeltrioxid wurde durch Erhitzen aus einer entsprechenden Menge 65-gewichtsprozentigen Oleums ausgetrieben, mit Stickstoff auf eine Konzentration von 5 Vol.-% verdünnt und innerhalb von 30 min in das Ausgangsprodukt eingeleitet. Das rohe Sulfierprodukt wurde im Anschluß bei einer Temperatur von 80 °C mit 214 g einer 20-gewichtsprozentigen wäßrigen Natriumhydroxidlösung neutralisiert und gleichzeitig hydrolysiert. Anschließend wurde das Reaktionsprodukt mit Natronlauge auf einen pH-Wert von 10 eingestellt. Man erhielt ein pastenförmiges Produkt mit folgenden Kennzahlen:
- Aniontensidgehalt :: 59,7 Gew.-%
- Alkenylsulfatanteil : 27,5 Gew.-%
- Alkylsulfatanteil : 20,7 Gew.-%
- Sulfonatanteil : 11,5 Gew.-%
- Unsulfierte Anteile :: 2,0 Gew.-%
- Natriumsulfat :: 2,8 Gew.-%
- Wasser :: 35,5 Gew.-%

Der Aniontensidgehalt und die unsulfierten Anteile wurden nach DGF-Einheitsmethoden, Stuttgart, 1950-1984, H-III-10 bzw. G-II-6b ermittelt. Der Sulfatgehalt wurde als Natriumsulfat berechnet, die Bestimmung des Wassergehalts erfolgte nach der Fischer Methode.

### Beispiel 2

Durch Zugabe der in der nachfolgenden Tabelle angegebenen Mengen des Produktes gemäß Beispiel 1 zu einer 55-gewichtsprozentigen wäßrigen Paste eines gesättigten C_{16/18}-Alkylsulfats (Sulfopon^{(R)} T 55, Hersteller Henkel) und Vermischen wurden die in der nachfolgenden Tabelle charakterisierten Pasten P1 bis P3 gemäß der Erfindung hergestellt. Die Messungen der Fließgrenzen und der Viskositäten (bei einer Scherrate D=10 s⁻¹) der so hergestellten Pasten wurden bei 60 °C mit einem schubspannungskontrollierten Rotationsrheometer (Carri-Med^{(R)} CS 100) mit Platte-Platte-Meßsystem durchgeführt. Zum Vergleich sind die auf gleiche Weise bestimmten Werte einer Alkenylsulfat-freien Lösung (V1) angegeben.

**Tabelle:**

| Alkenyl- und Alkylsulfatgehalte [Gew.-% Aktivsubstanz] und Fließdaten der Pasten | | | | |
|---|---|---|---|---|
| | **P1** | **P2** | **P3** | **V1** |
| Alkenylsulfat^{a)} | 6,88 | 13,75 | 20,62 | - |
| Alkylsulfat^{b)} | 46,43 | 37,85 | 29,28 | 55 |
| Fließgrenze [Pa] | 105 | 60 | 25 | 138 |
| Viskosität [Pa.s] | 19 | 12 | 7,5 | 32 |

| | | | | |
|---|---|---|---|---|
| ^{a)} aus entsprechenden Mengen des Produktes gemäß Beispiel 1 | | | | |
| ^{b)} Summe aus Na-C_{16/18}-Alkylsulfat (Sulfopon^{(R)} T 55, Hersteller Henkel) und Alkylsulfat aus dem Produkt gemäß Beispiel 1 | | | | |

## Patentansprüche

1. Verfahren zur Herstellung einer fließfähigen und pumpbaren wäßrigen Alkylsulfatpaste mit einem Feststoffgehalt von 50 bis 80 Gew.-%, dadurch gekennzeichnet, daß man eine wäßrige Paste, die 50 Gew.-% bis 80 Gew.-% eines Alkylsulfats der Formel I,
R¹-O-SO₃M¹ (I)
in der R¹ einen linearen oder verzweigtkettigen Alkylrest mit 12 bis 22 C-Atomen und M¹ ein Alkalimetall-, Ammonium-, Alkylammonium- oder Hydroxyalkylammonium-Ion bedeuten, mit einem Alkenylsulfat der Formel II,
R²-O-SO₃M² (II)
in der R² einen Alkenylrest mit 16 bis 22 C-Atomen und 1, 2 oder 3 Doppelbindungen und M² ein Alkalimetall-, Ammonium-, Alkylammonium- oder Hydroxyalkylammonium-Ion bedeuten, im Gewichtsverhältnis Alkylsulfat gemäß Formel I zu Alkenylsulfat gemäß Formel II von 10:1 bis 1:3 mischt.

2. Verfahren nach Anspruch 1, dadurch gekennzeichnet, daß man Alkenylsulfat gemäß Formel II und Alkylsulfat gemäß Formel I im Gewichtsverhältnis von Alkylsulfat zu Alkenylsulfat von 7:1 bis 1:2 mischt.

3. Verfahren nach Anspruch 1 oder 2, dadurch gekennzeichnet, daß man eine wäßrige Paste, welche Alkenylsulfat gemäß Formel II enthält, in einer solchen Menge mit einer wäßrigen Paste, die Alkylsulfat gemäß Formel I enthält, mischt, daß die Summe von Alkylsulfat und Alkenylsulfat in der resultierenden wäßrigen Paste 45 Gew.-% bis 80 Gew.-%, insbesondere 50 Gew.-% bis 75 Gew.-% beträgt und die resultierende Paste 20 Gew.-% bis 55 Gew.-%, insbesondere 25 Gew.-% bis 50 Gew.-% Wasser enthält.

4. Verfahren nach Anspruch 3, dadurch gekennzeichnet, daß die wäßrige Alkenylsulfat-Paste nicht mehr als 70 Gew.-%, insbesondere 2 Gew.-% bis 60 Gew.-% Alkylsulfat enthält.

## Claims

1. A process for the production of a free-flowing, pumpable water-containing alkyl sulfate paste with a solids content of 50 to 80% by weight, characterized in that an aqueous paste containing 50% by weight to 80% by weight of an alkyl sulfate corresponding to formula I:
R¹-O-SO₃M¹ (I)
in which R¹ is a linear or branched C₁₂₋₂₂ alkyl radical and M¹ is an alkali metal, ammonium, alkyl ammonium or hydroxyalkyl ammonium ion,
is mixed with an alkenyl sulfate corresponding to formula II:
R²-O-SO₃M² (II)
in which R² is an alkenyl radical containing 16 to 22 carbon atoms and 1, 2 or 3 double bonds and M² is an alkali metal, ammonium, alkyl ammonium or hydroxyalkyl ammonium ion,
in a ratio by weight of alkyl sulfate corresponding to formula I to alkenyl sulfate corresponding to formula II of 10:1 to 1:3.

2. A process as claimed in claim 1, characterized in that the alkenyl sulfate corresponding to formula II and the alkyl sulfate corresponding to formula I are mixed in a ratio by weight of alkyl sulfate to alkenyl sulfate of 7:1 to 1:2.

3. A process as claimed in claim 1 or 2, characterized in that a water-containing paste containing an alkenyl sulfate corresponding to formula II is mixed with a water-containing paste containing an alkyl sulfate corresponding to formula I in such a quantity that the sum total of alkyl sulfate and alkenyl sulfate in the resulting aqueous paste is 45% by weight to 80% by weight and more particularly 50% by weight to 75% by weight and the resulting paste contains 20% by weight to 55% by weight and more particularly 25% by weight to 50% by weight of water.

4. A process as claimed in claim 3, characterized in that the water-containing alkenyl sulfate paste contains no more than 70% by weight and, more particularly, between 2% by weight and 60% by weight of alkyl sulfate.

## Revendications

1. Procédé de fabrication d'une pâte aqueuse coulante et pompable de sulfate d'alkyle avec une concentration en matières solides comprise entre 50 et 80 % en poids, caractérisé en ce que l'on mélange une pâte aqueuse, qui contient 50 à 80 % en poids d'un sulfate d'alkyle de la formule I,
R¹-O-SO₃M¹ (I)
dans laquelle R¹ représente un radical alkyle à chaîne droite ou ramifiée, comportant 12 à 22 atomes de C, et M¹ est un ion de métal alcalin, d'ammonium, d'alkylammonium ou d'hydroxyalkylammonium, avec un sulfate d'alcényle de la formule II
R²-O-SO₃M² (II)
dans laquelle R² représente un radical alcényle à chaîne droite ou ramifiée, comportant 12 à 22 atomes de C, et M² est un ion de métal alcalin, d'ammonium, d'alkylammonium ou d'hydroxyalkylammonium, dans un rapport pondéral entre le sulfate d'alkyle selon la formule I et le sulfate d'alcényle selon la formule II, de 10:1 à 1:3.

2. Procédé selon la revendication 1, caractérisé en ce que l'on mélange un sulfate d'alcényle selon la revendication II et un sulfate d'alkyle selon la formule I, dans un rapport pondéral entre le sulfate d'alkyle et le sulfate d'alcényle de 7:1 à 1:2.

3. Procédé selon la revendication 1 ou 2, caractérisé en ce que l'on mélange une pâte aqueuse, qui contient le sulfate d'alcényle selon la formule II avec une pâte aqueuse, qui contient le sulfate d'alkyle selon la formule I, dans une proportion telle que la somme du sulfate d'alkyle et du sulfate d'alcényle dans la pâte aqueuse résultante atteint 45 à 80 % en poids, en particulier 50 à 75 % en poids, et que la pâte résultante contient 20 à 55 % en poids, en particulier 25 à 50 % en poids d'eau.

4. Procédé selon la revendication 3, caractérisé en ce que la pâte aqueuse de sulfate d'alcényle ne contient pas plus de 70 % en poids, en particulier de 2 à 60 % en poids de sulfate d'alkyle.
